# NOUVEAU FASCICULE DE BREVET EUROPEEN

(11) **EP 1 224 000 B2**
(45) Date de publication et mention de la décision concernant l'opposition: **04.07.2007**
(45) Mention de la délivrance du brevet: 31.03.2004
(21) Numéro de dépôt: 00974569.6
(22) Date de dépôt: 26.10.2000
(51) Int. Cl.: A61M 5/32, A61M 5/50

(54) **DISPOSITIF DE SECURITE POUR SERINGUE D'INJECTION**
SICHERHEITSVORRICHTUNG FÜR INJEKTIONSSPRITZEN
SAFETY DEVICE FOR INJECTION SYRINGE

(30) Priorité: 26.10.1999 FR 9913348
(43) Date de publication de la demande: 24.07.2002
(73) Titulaire: Rexam Pharma La Verpillière, 38290 La Verpilliere Cedex (FR)
(72) Inventeur: POUGET, Michel, F-38300 Domarin (FR); BONACCI, Fabrice, F-69800 Saint Priest (FR)
(74) Mandataire: de la Bigne, Guillaume Michel Marie
(86) Numéro de dépôt international: PCT/FR2000/002986
(87) Numéro de publication internationale: WO 2001/030427

(56) Documents cités:
- EP-A- 0 966 983
- EP-A- 1 090 653
- WO-A-00/76565
- WO-A-01/24856
- WO-A-98/35714
- FR-A- 2 794 650
- US-A- 5 562 626

## Description

La présente invention concerne un dispositif de sécurité pour seringue d'injection.

On sait que certains produits médicamenteux injectables sont distribués par doses dans des corps de seringues remplis à l'avance, auxquels il suffit d'ajouter des tiges de pistons et des aiguilles pour les transformer en seringues.

On sait par ailleurs, que les seringues constituent des instruments dangereux, notamment pour le personnel médical, du fait qu'après utilisation, leurs aiguilles, souillées et potentiellement contaminantes, peuvent accidentellement toucher, voire piquer, des personnes et les contaminer.

La demande WO 98/35714 décrit un dispositif de sécurité pour une seringue, comportant un poussoir pourvu de pattes de retenue aptes à coopérer avec un bourrelet annulaire réalisé à une extrémité du fourreau de protection. Un ressort hélicoïdal est disposé entre un épaulement intérieur du fourreau de protection et la collerette de la seringue. En fin d'injection, les pattes de retenue du poussoir sont écartées afin de permettre au fourreau de protection de venir recouvrir l'aiguille de la seringue.

Le brevet US 5 562 626 décrit un dispositif de sécurité pour une seringue, comportant un fourreau de protection avec à une extrémité des pattes élastiques aptes à s'appliquer sur une collerette de la seringue avant injection. Un ressort hélicoïdal est disposé entre un épaulement intérieur du fourreau de protection et la collerette de la seringue.

La présente invention vise à fournir un dispositif de sécurité grâce auquel ce genre d'accident ne peut pas se produire.

La présente invention a pour objet un dispositif de sécurité selon la revendication 1.

Grâce au dispositif de sécurité selon l'invention, le produit contenu dans la seringue peut être injecté à un patient de la même manière que si la seringue était utilisée seule, tant que le corps de la seringue se trouve en position d'injection, position dans laquelle l'aiguille montée sur le porte-aiguille de la seringue dépasse du fourreau.

Lorsque le piston arrive au voisinage du porte-aiguille, c'est-à-dire en fin d'injection du produit, le mécanisme de neutralisation agit sur les moyens de verrouillage et libère le corps de la seringue, lequel, sous la poussée du ressort, coulisse dans le fourreau pour atteindre sa position de sécurité dans laquelle le porte-aiguille se trouve en retrait et l'aiguille ne dépasse plus du fourreau.

Dans cette position de sécurité, que le corps de la seringue n'atteint qu'à la fin de l'injection, tout risque de blessure accidentelle par contact avec l'aiguille souillée est évité car l'aiguille, rétractée dans le fourreau, n'est plus accessible.

Le dispositif selon l'invention est particulièrement adapté à l'usage de seringues pré-remplies car le corps de la seringue se met en place dans le fourreau alors que le ressort est maintenu à l'état comprimé par les moyens de rétention.

En d'autres termes, l'utilisateur n'a pas à comprimer le ressort lorsqu'il engage le corps de la seringue dans le fourreau.

L'utilisation du dispositif selon l'invention s'en trouve grandement facilitée.

L'absence d'effort à fournir pour comprimer le ressort au moment de l'insertion du corps de la seringue permet en outre d'automatiser le montage du corps de seringues pré-remplies dans des dispositifs de sécurité selon l'invention.

Dans un mode de réalisation particulier de l'invention, le ressort hélicoïdal est logé à l'intérieur du fourreau dans une partie élargie de ce dernier, autour du corps de la seringue, et prend appui d'une part, sur une portée du fourreau, d'autre part, sur une collerette du corps de la seringue.

Dans ce mode de réalisation, le mécanisme de neutralisation est constitué par un capuchon coulissant dans le fourreau qui se déplace dans le fourreau en direction de la patte élastique pour l'écarter radialement en libérant le corps de seringue et/ou le ressort.

Le déplacement du capuchon s'effectue lorsque le piston arrive au voisinage du porte-aiguille dans le corps de la seringue.

Ce résultat est obtenu en dimensionnant le fourreau de manière que le doigt de l'utilisateur arrive au contact de la bague en fin de course du piston, c'est-à-dire à la fin de l'injection.

Ainsi, en bout de course de la tige de piston, l'utilisateur enfonce le capuchon, lequel écarte la patte élastique, laquelle libère le corps de la seringue, lequel remonte dans le fourreau et rétracte ainsi l'aiguille dans ledit fourreau.

Dans le but de mieux faire comprendre l'invention, on va en décrire maintenant deux modes de réalisation donnés à titre d'exemples non limitatifs, en référence au dessin annexé dans lequel :
- la figure 1 est une vue en coupe axiale d'un dispositif de sécurité,
- la figure 2 est une vue en élévation d'une seringue,
- la figure 3 est une vue en perspective et en coupe selon III-III de la figure 1,
- la figure 4 est une vue analogue à la figure 3, montrant la seringue en cours d'introduction dans le fourreau,
- la figure 5 est une vue analogue à la figure 4, montrant la seringue engagée dans le fourreau,
- la figure 6 est une vue analogue à la figure 5, montrant un capuchon du fourreau en cours de mise en place sur le fourreau,
- la figure 7 est une vue rapprochée de la partie supérieure du fourreau muni du capuchon,
- la figure 8 est une vue en perspective du dispositif prêt à l'emploi,
- la figure 9 est une vue analogue à la figure 7, montrant le dispositif lorsque la tige de piston arrive en fin de course sous la pression exercée par le doigt d'un utilisateur,
- la figure 10 est une vue en coupe selon X-X de la figure 9, montrant le dispositif lorsque le doigt de l'utilisateur relâche la pression,
- la figure 11 est une vue en coupe analogue à la figure 10, montrant le dispositif après utilisation, et

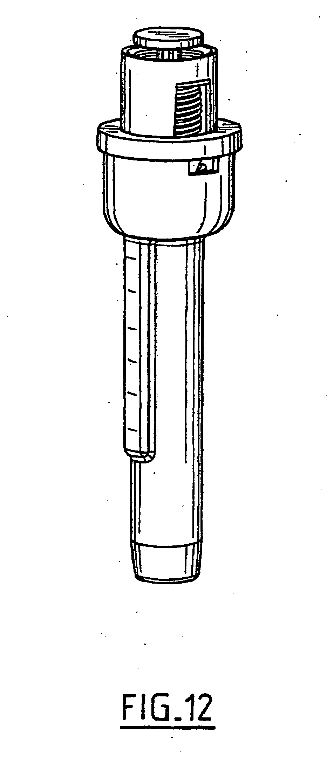

- la figure 12 est une vue en perspective montrant le dispositif après utilisation.

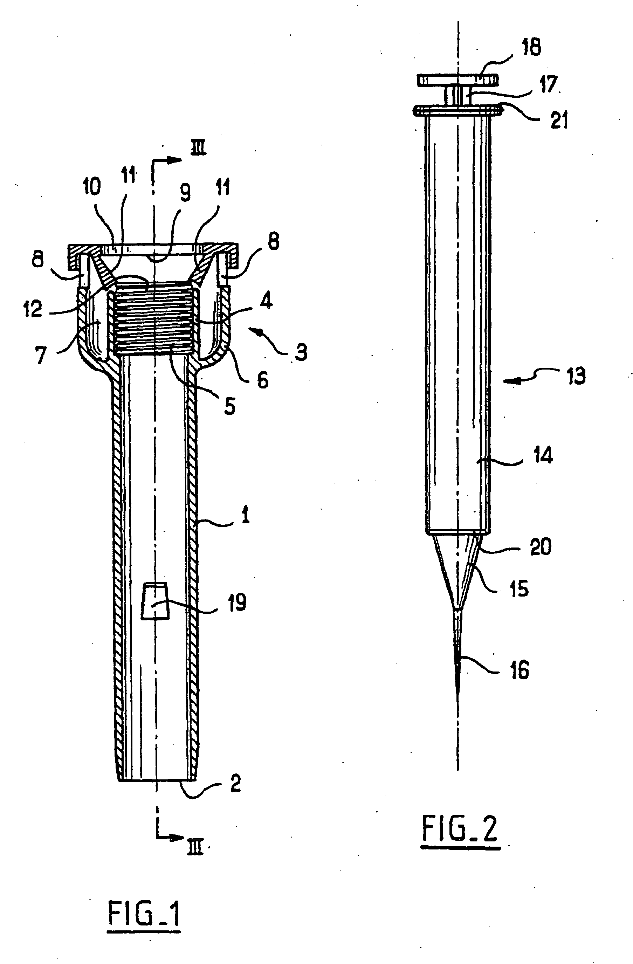

Le fourreau du dispositif de sécurité représenté à la figure 1 comprend une partie cylindrique 1 comportant une ouverture inférieure 2 et, à l'opposé de cette ouverture 2, une tête 3 constituée par une portion tubulaire 4 qui contient un ressort hélicoïdal 5 prolongeant la cavité intérieure de la partie cylindrique 1 et par une paroi extérieure 6 qui double la portion tubulaire 4 et définit autour d'elle une chambre annulaire 7.

La paroi 6 comporte deux encoches 8 diamétralement opposées qui partent de son bord supérieur 9.

Ce bord 9 est recouvert par une bague 10 qui est encliquetée ou collée ou soudée sur la tête. La bague 10 a été réalisée séparément pour des raisons de fabrication mais pourrait être obtenue d'un seul tenant avec le fourreau.

La bague 10 supporte deux pattes déformables 11 qui s'étendent depuis le voisinage du bord 9 en direction du bord supérieur 12 de la portion tubulaire 4.

L'extrémité inférieure des pattes 11 est concave de manière à servir de butée au ressort hélicoïdal 5 qui est logé dans la portion tubulaire 4 et qui a été préalablement comprimé axialement.

La seringue 13, qui est représentée à la figure 2, comprend un corps 14, un porte-aiguille 15 portant une aiguille 16, un piston mobile non représenté qui est apte à coulisser dans le corps 14 et une tige de piston 17 qui dépasse à la partie supérieure de la seringue et se termine par un poussoir 18 sur lequel un utilisateur peut exercer une force pour procéder à une injection.

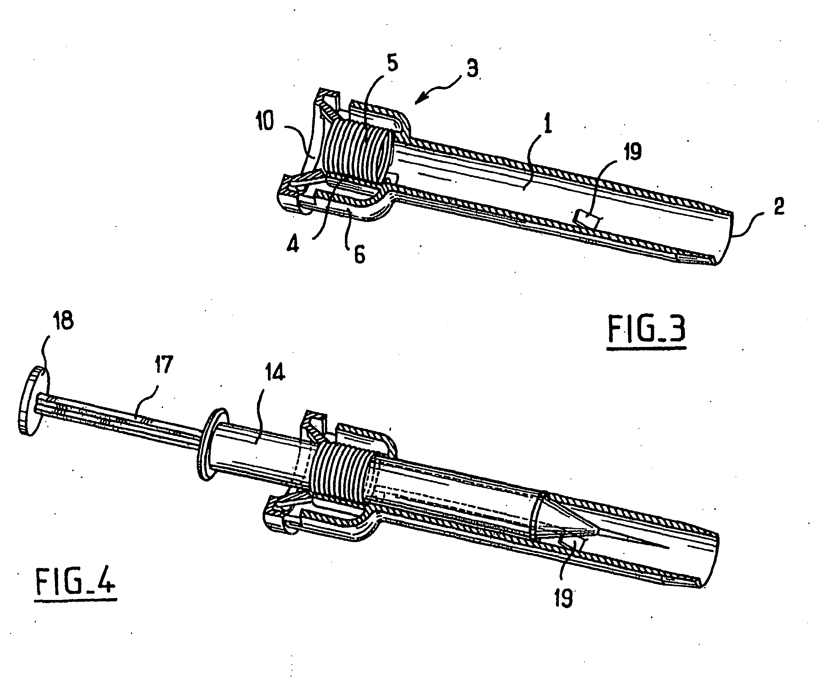

La seringue 13 se met en place dans le fourreau de la figure 1 comme représenté à la figure 4.

Du fait que le ressort hélicoïdal 5 prolonge la paroi intérieure de la partie cylindrique 1 du fourreau, le corps de la seringue, dont le diamètre externe et légèrement inférieur au diamètre interne de la partie cylindrique 1 du fourreau, pénètre sans difficulté à l'intérieur du ressort et vient se loger dans le fourreau.

Ce dernier comporte, en saillie de la paroi intérieure de sa partie cylindrique 1, une patte élastique 19 qui constitue une butée d'arrêt sur l'épaulement constitué par l'extrémité inférieure 20 du corps 14 de la seringue.

Moyennant un effort relativement important, la butée 19 peut s'effacer et laisser passer le corps de la seringue.

Lorsque le bord supérieur 21 du corps 14 de la seringue, qui forme une collerette, arrive au contact des pattes élastiques de la tête du fourreau et écarte ces pattes élastiques, il libère le ressort sur une faible longueur, le ressort venant alors prendre appui contre la collerette 21 de la seringue qui se situe au voisinage immédiat du ressort.

Lorsque la seringue continue sa progression en direction de la position d'injection, la collerette écarte davantage les pattes élastiques 11 et passe à son tour en dessous de leurs extrémités inférieures en comprimant à nouveau le ressort dans la portion tubulaire 4.

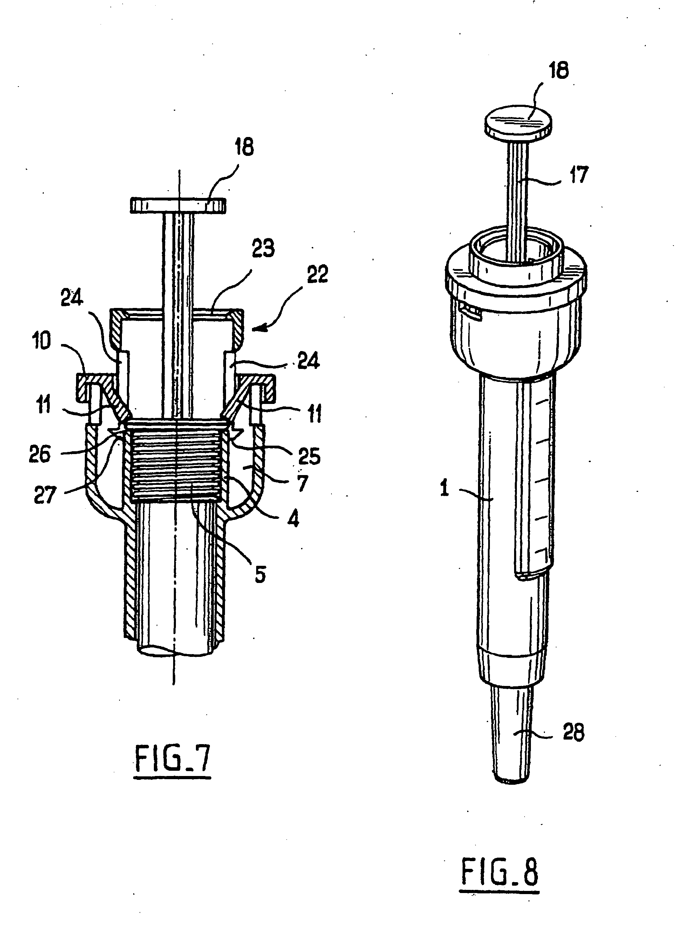

Les pattes élastiques 11 reviennent en position au-dessus de la collerette, comme on le voit à la figure 7, et bloquent la seringue en position d'injection dans le fourreau.

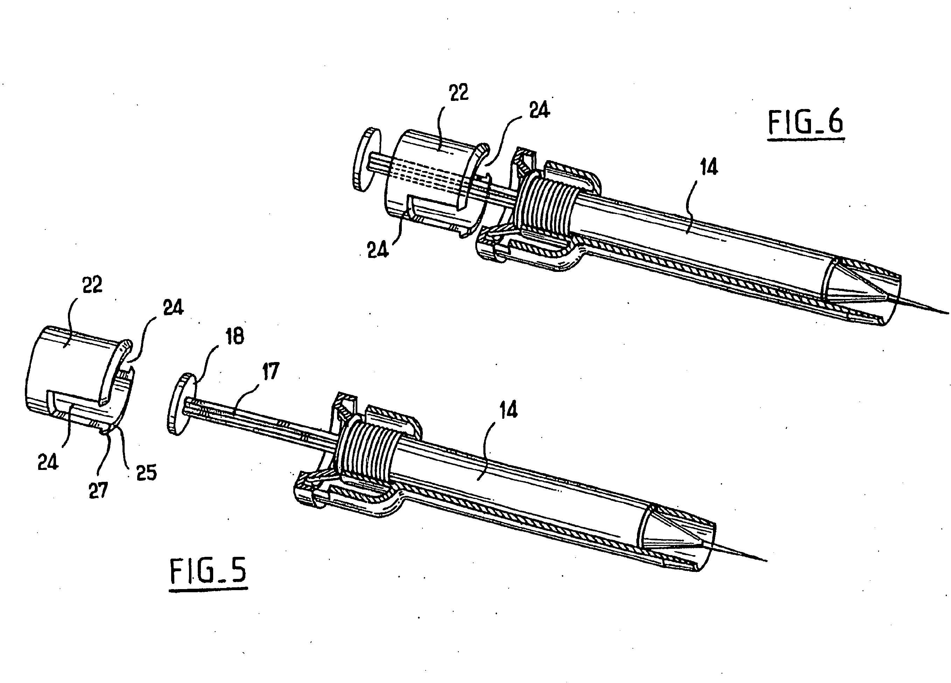

Dans cette position d'injection, le porte-aiguille 15 affleure à l'extrémité inférieure 2 du fourreau, comme on le voit notamment aux figures 5 et 6.

Un capuchon percé 22 est ensuite engagé dans la tête du fourreau. Ce capuchon 22 possède une forme générale cylindrique et un diamètre qui lui permet de se loger dans la chambre annulaire 7.

Il comporte un perçage central 23 qui laisse le passage au poussoir 18 de la seringue, ainsi que deux encoches latérales 24 qui partent de son bord inférieur 25 et lui permettent de s'engager de part et d'autre des deux pattes élastiques 11.

Comme on le voit à la figure 7, le diamètre externe du capuchon correspond au diamètre interne de la bague 10, de sorte que la collerette 26, dirigée vers l'extérieur, qui est formée sur le bord inférieur 25 du capuchon, ne peut pénétrer dans la tête que par déformation vers l'intérieur du capuchon, grâce au chanfrein 27 prévu sur la collerette, mais ne peut, après insertion, sortir de la tête.

Le capuchon ainsi mis en place s'engage partiellement autour de la portion tubulaire 4 et demeure libre dans la tête 3.

Le dispositif ainsi préparé, qui est représenté à la figure 8, est prêt à l'emploi.

L'aiguille de la seringue, qui fait saillie à l'extrémité inférieure du capuchon, peut être protégée par un bouchon 28.

Pour injecter le produit contenu dans la seringue, l'utilisateur enfonce le poussoir 18 pour déplacer le piston mobile du corps de la seringue.

Du fait que la collerette 21 du corps de la seringue s'appuie sur le bord supérieur 12 de la portion tubulaire 4, le corps de la seringue ne peut pas descendre dans le fourreau et la pression exercée sur le poussoir 18 se traduit par l'enfoncement de la tige de piston 17.

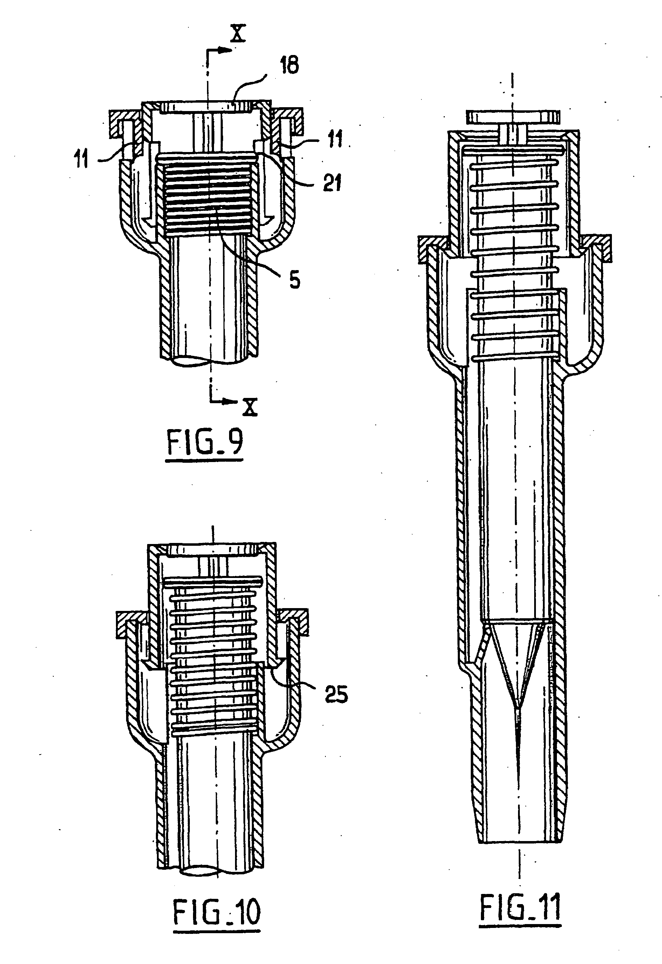

Au cours de cet enfoncement, le poussoir 18 arrive à hauteur du capuchon 22 et pénètre dans son perçage 23, comme on le voit à la figure 9.

A partir de cet instant, l'action de l'utilisateur s'exerce non seulement sur le poussoir 18 mais également sur le capuchon 22, lequel s'enfonce dans la tête du fourreau en même temps que le poussoir.

Cette action a pour effet d'amener la partie dépourvue d'encoche du capuchon 22 à hauteur des pattes élastiques 11.

Il en résulte que le capuchon exerce sur les deux pattes élastiques 11 une force dirigée vers l'extérieur qui les écarte, libérant ainsi la collerette 21 et le ressort hélicoïdal 5.

Le corps de la seringue n'est alors plus maintenu en position que par l'action de l'utilisateur sur le poussoir.

Lorsque l'utilisateur relâche la pression exercée sur le poussoir, le ressort hélicoïdal se détend et repousse le corps de la seringue ainsi que le poussoir 18 et le capuchon 22 vers le haut, comme on le voit à la figure 10, jusqu'à atteindre une position de sécurité représentée à la figure 11 dans laquelle la collerette 26 du capuchon 22 arrive en butée contre la bague 10.

Simultanément, l'épaulement constitué par le bord inférieur 20 du corps de la seringue passe au-dessus de la patte 19, de sorte que la seringue se trouve empêchée de s'enfoncer à nouveau dans le fourreau.

La seringue atteint de cette manière sa position de sécurité dans laquelle le porte-seringue se trouve en retrait dans le fourreau et l'aiguille ne dépasse plus à l'extrémité inférieure 2 du fourreau.

On prévient ainsi tout risque de contact accidentel avec l'aiguille.

On voit que le dispositif selon l'invention procure une bonne sécurité vis-à-vis du problème des contaminations accidentelles.

En outre, il apparaît clairement que la précontrainte du ressort facilite l'introduction de la seringue dans le fourreau, en permettant notamment une automatisation de cette mise en place.

En particulier, le dispositif de sécurité peut être livré avec son ressort précontraint à un utilisateur de seringue qui pourra facilement mettre en place ses seringues pré-remplies dans le dispositif de sécurité, sans avoir à leur apporter la moindre modification.

Il est bien entendu que le mode de réalisation qui vient d'être décrit ne présente aucun caractère limitatif et qu'il pourra recevoir toutes modifications désirables sans sortir pour cela du cadre de l'invention.

## Revendications

1. Dispositif de sécurité pour une seringue constituée d'un corps (14), d'un porte-aiguille (15), monté à une extrémité du corps, d'un piston axialement mobile dans le corps et d'une tige de piston (17) dépassant du corps à l'opposé du porte-aiguille et apte à pousser le piston dans le corps en direction du porte-aiguille, le dispositif comprenant :
- un fourreau (1) dans lequel un corps de seringue peut coulisser axialement entre une position d'injection dans laquelle le porte-aiguille affleure à une extrémité (2) du fourreau et une position de sécurité dans laquelle le porte-aiguille se trouve en retrait dans le fourreau, le fourreau (1) ayant une tête (3) à l'opposé de l'extrémité (2)
- un ressort hélicoïdal (5) qui est axialement comprimé lorsque le corps de la seringue se trouve en position d'injection, ledit ressort exerçant alors sur ledit corps une force tendant à le faire coulisser en position de sécurité,
- des moyens de rétention (11) du corps de la seringue en position d'injection, et
- un mécanisme (22) de neutralisation des moyens de rétention (11) agissant sur lesdits moyens de rétention lorsque la tige de piston de la seringue a poussé le piston dans le corps jusqu'au voisinage du porte-aiguille,
**caractérisé par le fait que**
- les moyens de rétention (11) du corps de la seringue en position d'injection dans le fourreau (1), sont constitués par au moins une patte élastique (11) à débattement radial, s'étendant sensiblement longitudinalement au fourreau, et agencée pour maintenir le ressort (5) à l'état comprimé même lorsque le corps (14) de la seringue ne se trouve pas dans le fourreau (1),
- la patte étant supportée par une bague encliquetée ou collée ou soudée sur la tête ou d'un seul tenant avec le fourreau, et
- le mécanisme de neutralisation est constitué par un capuchon (22) coulissant dans le fourreau, qui peut se déplacer dans le fourreau en direction de la patte élastique (11) pour l'écarter radialement en libérant le corps de seringue et/ou le ressort.

2. Dispositif selon la revendication 1, **caractérisé par le fait que** le ressort hélicoïdal (5) est logé à l'intérieur du fourreau (1) dans une partie élargie de ce dernier, autour du corps (14) de la seringue et prend appui d'une part sur une portée du fourreau, et d'autre part sur une collerette (21) du corps de la seringue.

## Claims

1. A safety device for a syringe constituted by a body (14), a needle carrier (15) mounted at one end of the body, a piston that is axially movable in the body, and a piston plunger (17) projecting from the body at its end opposite from the needle carrier and suitable for pushing the piston inside the body towards the needle carrier, the device comprising :
- a sheath (1) in which the syringe body can slide axially between an injection position in which the needle carrier is flush with one end (2) of the sheath and a safe position in which the needle carrier is retracted into the sheath, the sheath (1) having a head (3) opposite to the end (2),
- a helical spring (5) which is axially compressed when the syringe body is in the injection position, said spring then exerting on said body a force tending to cause it to slide into the safe position,
- retention means (11) for retaining the syringe body in the injection position, and
- a release mechanism (22) for releasing the retention means (11) acting on said retention means when the piston plunger of the syringe has pushed the piston inside the body into the vicinity of the needle carrier,
**characterized by** the fact that,
- the retention means (11) for retaining the body of the syringe in the injection position in the sheath (1) are constituted by at least one radially-movable resilient tab (11) extending substantially longitudinally relative to the sheath and arranged to hold the spring in the compressed state even when the syringe body (14) is not in the sheath (1), the tab being supported by a ring snap-fastened or stuck or bonded to the head or integrally with the sheath, and
- the release mechanism being constituted by a cap (22) sliding in the sheath and which moves inside the sheath towards the resilient tab (11) to move it radially away so as to release the syringe body and/or the spring.

2. A device according to claim 1, **characterized by** the fact that the helical spring (5) is received inside the sheath (1) in an enlarged portion thereof, around the syringe body (14), and it bears both against a bearing surface of the sheath and against a collar (21) of the syringe body.

## Patentansprüche

1. Sicherheitsvorrichtung für eine Injektionsspritze, die ein Gehäuse (14), einen Nadelträger (15), der an einem Endbereich (2) des Gehäuses angeordnet ist, einen axial in diesem Gehäuse verschiebbaren Kolben und eine Kolbenstange (17) aufweist, welche entgegengesetzt dem Nadelträger aus dem Gehäuse vorsteht und ausgelegt ist, den Kolben in Richtung des Nadelträgers in das Gehäuse zu drücken, diese Sicherheitsvorrichtung hat eine Stulpe (1), in der ein Gehäuse der Injektionsspritze axial zwischen einer Injektionsstellung, in welcher der Nadelträger an einem Endbereich (2) der Stulpe vorsteht, und einer Sicherheitsstellung, in welcher der Nadelträger sich zurückgezogen in der Stulpe befindet, axial verschiebbar ist, wobei die Stulpe (1) an dem Gegenendbereich einen Kopf (3) hat; eine Schraubenfeder (5), die axial komprimiert ist, wenn sich das Gehäuse der Injektionsspritze in der Injektionsstellung befindet, wobei diese Feder dann das Gehäuse mit einer Kraft belastet, die es in die Sicherheitsstellung zu schieben trachtet; Ruckhaltemittel (11) des Gehäuses der Injektionsspritze in der Injektionsstellung und einen Mechanismus (22) für die Neutralisierung dieser Rückhaltemittel (11), welcher auf die Rückhaltemittel einwirkt, wenn die Kolbenstange der Injektionsspritze den Kolben in dem Gehäuse bis in die Nähe des Nadelträgers gedrückt hat, **dadurch gekennzeichnet, dass** die Rückhaltemittel (11) des Gehäuses der Injektionsspritze in Injektionsstellung in der Stulpe (1) durch mindestens einen radial auslenkbaren, elastischen Lappen (11) gebildet sind, der sich im Wesentlichen in Längsrichtung der Stulpe erstreckt und ausgebildet ist, die Feder (5) im komprimierten Zustand zu halten, auch wenn das Gehäuse (14) der Injektionsspritze sich nicht in der Stulpe (1) befindet, wobei der Lappen durch einen an dem Kopf eingerastet oder geklebt oder verschweißt oder mit der Stulpe solidarisiert Ring verträgt ist, und der Mechanismus (22) für die Neutralisierung durch eine Kappe (22) gebildet ist, die in der Stulpe (1) gleitet und die sich in der Stulpe (1) in Richtung des elastischen Lappens (11) verschiebt, um diesen radial zu spreizen und das Gehäuse der Injektionsspritze und/oder die Feder freizugeben.

2. Vorrichtung nach Anspruch 1, **dadurch gekennzeichnet, dass** die Schraubenfeder (5) im Inneren der Stulpe (1) in einem vergrößerten Bereich dieser Stulpe (1) und um das Gehäuse (14) der Injektionsspritze befindet und sich einerseits an einem Bereich der Stulpe und andererseits an einem Kragen (21) des Gehäuse der Injektionsspritze abstutzt.
